# EUROPEAN PATENT APPLICATION

(11) **EP 3 950 927 A1**
(43) Date of publication of application: **09.02.2022**
(21) Application number: 20781770.1
(22) Date of filing: 01.04.2020
(51) Int. Cl.: C12N 1/15, C12P 21/02

(54) **METHOD FOR PRODUCING RECOMBINANT PROTEIN**

(30) Priority: 02.04.2019 JP 2019070585
(71) Applicant: Spiber Inc., Tsuruoka-shi, Yamagata 997-0052 (JP)
(72) Inventor: KOBAYASHI Masato, Tsuruoka-shi, Yamagata 997-0052 (JP); KURACHI Kenji, Tsuruoka-shi, Yamagata 997-0052 (JP); MORI Eiji, Tsuruoka-shi, Yamagata 997-0052 (JP); NODA Takanobu, Tsuruoka-shi, Yamagata 997-0052 (JP)
(74) Representative: Handley, Matthew Edward
(86) International application number: PCT/JP2020/015094
(87) International publication number: WO 2020/204102

(57) **Abstract**

The present invention relates to a method for producing a recombinant protein, the method including: a first step of culturing a recombinant cell expressing a recombinant protein under control of an inducible promoter in a medium containing galactose and at least two selected from the group consisting of mannitol, raffinose, and melibiose; and a second step of further culturing the recombinant cell under a condition that expression from the inducible promoter is induced after the first step.

## Description

### Technical Field

The present invention relates to a method for producing a recombinant protein.

### Background Art

In industrial production of useful substances by microorganisms, improvement of productivity is an important issue. For this purpose, various culture methods have been studied. In the production of a recombinant protein, a method is generally used in which an expression cassette of a recombinant protein under the control of an inducible promoter is introduced into host cells, the recombinant cells are introduced into a culture medium, after the culture, the recombinant cells are grown to a desired concentration by feeding nutrients, and an inducing agent such as arabinose, lactose or isopropyl-β-thiogalactopyranoside (IPTG) is added according to the promoter to induce expression of the recombinant protein.

For example, when a T7 promoter is used for expression of a recombinant protein, an IPTG induction system pET system in combination with T7 RNA polymerase is known (Patent Literature 1 and Non Patent Literature 1).

Although the T7 promoter expression system is strong, expression of the recombinant protein may be a strong stress for the host, affecting cell growth and suppressing the increase in expression efficiency. An attempt has been made to improve the sequence of the promoter or the structure of a so-called transcription factor acting on the promoter by controlling the transcription level/response of gene (Non Patent Literatures 2 and 3). Although such improvement has been attempted, establishment of a system with higher productivity is required.

### Citation List

### Patent Literature

Patent Literature 1: US 4,952,496 B

### Non Patent Literature

Non Patent Literature 1: Methods Enzymol, 1990, 185, pp. 60 to 89
Non Patent Literature 2: Biotechnol Bioeng, 1991, 37, pp. 318 to 324
Non Patent Literature 3: PLoS Comput Biol., 2012, 12, e1002811

### Summary of Invention

### Technical Problem

An object of the present invention is to provide a method for producing a recombinant protein with high productivity.

### Solution to Problem

The present invention relates to, for example, the following inventions.
[1] A method for producing a recombinant protein, the method including:
   a first step of culturing a recombinant cell expressing a recombinant protein under control of an inducible promoter in a medium containing galactose and at least two selected from the group consisting of mannitol, raffinose, and melibiose; and
   a second step of further culturing the recombinant cell under a condition that expression from the inducible promoter is induced after the first step.
[2] The production method according to [1], in which the inducible promoter is an isopropyl-β-thiogalactopyranoside (IPTG) inducible promoter.
[3] The production method according to [1] or [2], in which the inducible promoter is a T7 promoter.
[4] The production method according to [2] or [3], in which the second step is a step of adding IPTG to the medium and further culturing the recombinant cell.
[5] The production method according to any one of [1] to [4], in which the medium contains galactose, mannitol, raffinose, and melibiose.
[6] The production method according to any one of [1] to [4], in which the medium contains galactose, mannitol, and raffinose.
[7] The production method according to any one of [1] to [6], in which the recombinant protein is a structural protein.
[8] The production method according to any one of [1] to [7], in which the recombinant protein is fibroin.
[9] The production method according to any one of [1] to [8], in which the recombinant protein is spider silk fibroin.
[10] The production method according to any one of [1] to [9], in which the recombinant cell is a bacillus.
[11] The production method according to any one of [1] to [10], in which the recombinant cell is a microorganism belonging to the genus *Escherichia.*

### Advantageous Effects of Invention

According to the present invention, it is possible to provide a method for producing a recombinant protein with high productivity.

### Description of Embodiments

Hereinafter, embodiments of the present invention will be described in detail. However, the present invention is not limited to the following embodiments.

A method for producing a recombinant protein according to the present embodiment includes: a first step of culturing a recombinant cell expressing a recombinant protein under control of an inducible promoter in a medium containing galactose and at least two selected from the group consisting of mannitol, raffinose, and melibiose; and a second step of further culturing the recombinant cell under a condition that expression from the inducible promoter is induced after the first step.

### (Recombinant protein)

Examples of the recombinant protein produced by the production method according to the present embodiment (hereinafter, may be referred to as "target protein") can include any protein that is preferably produced on an industrial scale, and examples thereof can include proteins that can be used for industrial purposes, proteins that can be used for medical purposes, and structural proteins. Specific examples of the protein that can be used for industrial purposes or medical purposes can include a spider silk protein, an enzyme, a regulatory protein, a receptor, a peptide hormone, a cytokine, a membrane or transport protein, an antigen used for vaccination, a vaccine, an antigen-binding protein, an immunostimulatory protein, an allergen, and a full length antibody or an antibody fragment or a derivative thereof. Specific examples of the structural protein can include fibroin (for example, spider silk fibroin (spider silk), silkworm silk, and the like), keratin, collagen, elastin, resilin, and a protein derived from these proteins.

As used herein, the fibroin includes naturally derived fibroin and modified fibroin. As used herein, the "naturally derived fibroin" refers to fibroin having an amino acid sequence identical to that of naturally derived fibroin, and the "modified fibroin" refers to fibroin having an amino acid sequence different from that of the naturally derived fibroin.

The fibroin may be spider silk fibroin. The "spider silk fibroin" includes natural spider silk fibroin and modified fibroin derived from the natural spider silk fibroin. Examples of the natural spider silk fibroin can include spider silk proteins (SSP) produced by arachnids.

The fibroin may be, for example, a protein having a domain sequence represented by Formula 1: [(A)ₙ motif-REP]ₘ or Formula 2: [(A)ₙ motif-REP]ₘ-(A)ₙ motif. An amino acid sequence (N-terminal sequence or C-terminal sequence) may be further added to either or both of the N-terminal side and the C-terminal side of the domain sequence of the fibroin according to the present embodiment. The N-terminal sequence and the C-terminal sequence are not limited thereto, but, typically are regions having no repetitions of amino acid motifs characterized in fibroin, and each consists of amino acids of approximately 100 residues.

The "domain sequence" as used herein is an amino acid sequence that produces a crystal region (typically, corresponding to the (A)ₙ motif of the amino acid sequence) and an amorphous region (typically, corresponding to the REP of the amino acid sequence) specific to fibroin, and means an amino acid sequence represented by Formula 1: [(A)ₙ motif-REP]ₘ or Formula 2: [(A)ₙ motif-REP]ₘ-(A)ₙ motif. Here, the (A)ₙ motif represents an amino acid sequence mainly consisting of alanine residues, and the number of amino acid residues therein is 2 to 27. The number of the amino acid residues in the (A)ₙ motif may be an integer of 2 to 20, 4 to 27, 4 to 20, 8 to 20, 10 to 20, 4 to 16, 8 to 16, or 10 to 16. In addition, the proportion of the number of alanine residues in the total number of the amino acid residues in the (A)ₙ motif may be 40% or more, or may also be 60% or more, 70% or more, 80% or more, 83% or more, 85% or more, 86% or more, 90% or more, 95% or more, or 100% (meaning that the (A)ₙ motif consists of only alanine residues). At least seven of a plurality of (A)ₙ motifs in the domain sequence may consist of only alanine residues. The REP represents an amino acid sequence consisting of 2 to 200 amino acid residues. The REP may be an amino acid sequence consisting of 10 to 200 amino acid residues. m represents an integer of 2 to 300, and may be an integer of 10 to 300. A plurality of (A)ₙ motifs may be the same amino acid sequences or different amino acid sequences. A plurality of REPs may be the same amino acid sequences or different amino acid sequences.

Examples of the naturally derived fibroin can include a protein having a domain sequence represented by Formula 1: [(A)ₙ motif-REP]ₘ or Formula 2: [(A)ₙ motif-REP]ₘ-(A)ₙ motif. Specific examples of the naturally derived fibroin can include fibroin produced by insects or arachnids.

Examples of the fibroin produced by insects can include silk proteins produced by silkworms such as Bombyx mori, Bombyx mandarina, Antheraea yamamai, Anteraea pernyi, Eriogyna pyretorum, Pilosamia Cynthia ricini, Samia cynthia, Caligura japonica, Antheraea mylitta, and Antheraea assama and a hornet silk protein secreted by larvae of Vespa simillima xanthoptera.

More specific examples of the fibroin produced by insects can include the silkworm fibroin L chain (GenBank Accession Nos. M76430 (nucleotide sequence) and AAA27840.1 (amino acid sequence)).

Examples of the fibroin produced by arachnids include spider silk proteins produced by spiders belonging to the order *Araneae.* More specific examples thereof include spider silk proteins produced by spiders belonging to the genus *Araneus,* such as *Araneus ventricosus, Araneus diadematus, Araneus pinguis, Araneus pentagrammicus,* and *Araneus nojimai*, spiders belonging to the genus *Neoscona,* such as *Neoscona scylla, Neoscona nautica, Neoscona adianta,* and *Neoscona scylloides,* spiders belonging to the genus *Pronus,* such as *Pronous minutus*, spiders belonging to the genus *Cyrtarachne,* such as *Cyrtarachne bufo* and *Cyrtarachne inaequalis*, spiders belonging to the genus *Gasteracantha,* such as *Gasteracantha kuhlii* and *Gasteracantha mammosa,* spiders belonging to the genus *Ordgarius,* such as *Ordgarius hobsoni* and *Ordgarius sexspinosus,* spiders belonging to the genus *Argiope,* such as *Argiope amoena, Argiope minuta,* and *Argiope bruennichi,* spiders belonging to the genus *Arachnura,* such as *Arachnura logio,* spiders belonging to the genus *Acusilas,* such as Acusilas coccineus, spiders belonging to the genus *Cytophora,* such as *Cyrtophora moluccensis, Cyrtophora exanthematica,* and *Cyrtophora unicolor,* spiders belonging to the genus *Poltys,* such as *Poltys illepidus,* spiders belonging to the genus *Cyclosa,* such as *Cyclosa octotuberculata*, *Cyclosa sedeculata, Cyclosa vallata,* and *Cyclosa atrata,* and spiders belonging to the genus *Chorizopes,* such as *Chorizopes nipponicus,* and spider silk proteins produced by spiders belonging to the family *Tetragnathidae,* such as spiders belonging to the genus *Tetragnatha,* such as *Tetragnatha praedonia, Tetragnatha maxillosa, Tetragnatha extensa,* and *Tetragnatha squamata,* spiders belonging to the genus *Leucauge,* such as *Leucauge magnifica, Leucauge blanda,* and *Leucauge subblanda*, spiders belonging to the genus *Nephila,* such as *Nephila clavata* and *Nephila pilipes,* spiders belonging to the genus *Menosira,* such as *Menosira ornata,* spiders belonging to the genus *Dyschiriognatha,* such as *Dyschiriognatha tenera,* spiders belonging to the genus *Latrodectus,* such as *Latrodectus mactans, Latrodectus hasseltii, Latrodectus geometricus,* and *Latrodectus tredecimguttatus,* and spiders belonging to the genus *Euprosthenops.* Examples of the spider silk protein can include dragline silk proteins such as MaSps (MaSp1 and MaSp2) and ADFs (ADF3 and ADF4), MiSps (MiSp1 and MiSp2), AcSps, PySps, and Flag.

Examples of the keratin-derived protein can include a type I keratin of *Capra hircus*, and the like.

Examples of the collagen-derived protein can include a protein having a domain sequence represented by Formula 3: [REP2]ₒ (here, in Formula 3, o represents an integer of 5 to 300, REP2 represents an amino acid sequence consisting of Gly-X-Y, and X and Y represent an optional amino acid residue other than Gly, and a plurality of REP2s may be the same amino acid sequences or different amino acid sequences).

Examples of the elastin-derived protein can include proteins having the amino acid sequences of NCBI Genbank Accession Nos. AAC98395 (human), 147076 (sheep), NP786966 (cow), and the like.

Examples of the resilin-derived protein can include a protein having a domain sequence represented by Formula 4: [REP3]ₚ (here, in Formula 4, p represents an integer of 4 to 300, REP3 represents an amino acid sequence consisting of Ser-J-J-Tyr-Gly-U-Pro. J represents an optional amino acid residue and is particularly preferably an amino acid residue selected from the group consisting of Asp, Ser, and Thr, U represents an optional amino acid residue, and is particularly preferably an amino acid residue selected from the group consisting of Pro, Ala, Thr, and Ser, and a plurality of REP3s may be the same amino acid sequences or different amino acid sequences).

### (Recombinant cells expressing recombinant protein)

The recombinant cells according to the present embodiment express a recombinant protein under the control of an inducible promoter. The recombinant cells according to the present embodiment have, for example, a nucleic acid sequence encoding a recombinant protein, an inducible promoter sequence operably linked to the nucleic acid sequence, and as necessary, one or more regulatory sequences operably linked to the nucleic acid sequence (hereinafter, also referred to as "recombinant protein expression cassette").

The inducible promoter is only required to be an inducible promoter that functions in host cells and can induce expression of a recombinant protein. The inducible promoter is a promoter that can control transcription by presence of an inducer (expression inducer), absence of a repressor molecule, or physical factors such as increase or decrease in the temperature, osmotic pressure, pH value, or the like.

Specific examples of the inducible promoter in the case of using a prokaryote as a host include an IPTG inducible promoter (for example, a T7 promoter, tac and trc promoters, and lac and lacUV5 promoters) induced by lactose or IPTG which is an analog thereof.

The regulatory sequence is a sequence that controls expression of a recombinant protein in a host (for example, an enhancer, a ribosome binding sequence, and a transcription termination sequence), and can be appropriately selected depending on the type of the host. The type of the expression vector such as a plasmid vector, a viral vector, a cosmid vector, a fosmid vector, or an artificial chromosome vector can be appropriately selected depending on the type of the host.

The recombinant cells according to the present embodiment can be obtained, for example, by a method of transforming a host with an expression vector containing at least a nucleic acid sequence encoding a recombinant protein. The expression vector may have a nucleic acid sequence encoding a recombinant protein, an inducible promoter sequence operably linked to the nucleic acid sequence, and as necessary, one or more regulatory sequences operably linked to the nucleic acid sequence.

The recombinant cells according to the present embodiment may have the recombinant protein expression cassette outside the chromosome, or may have the recombinant protein expression cassette on the chromosome (on the genome).

A known method can be used as a method for transforming the host, and examples thereof can include transforming the host using a plasmid vector.

A known method can be used as the method for integrating the recombinant protein expression cassette into the genome. Examples thereof can include a λ red method to which a recombination mechanism in double-strand-break repair in λ phage is applied, a Red/ET homologous recombination method, and a transfer method utilizing a transposon activity in which pUT-mini Tn5 is used. For example, the recombinant protein expression cassette can be integrated into the genome of the host using the "kit for introducing gene by transposon: pUTmini-Tn5 Kit" of Biomedal, S.L. according to the method described in the kit. In this case, the recombinant protein expression cassette may be integrated into the genome of the host by causing recombination so that a DNA fragment containing at least the nucleic acid sequence encoding the recombinant protein is operably linked to one or more regulatory sequences on the genome of the host cells.

Both prokaryotes such as bacteria, and eukaryotes such as yeasts, filamentous fungi, insect cells, animal cells, and plant cells can be suitably used as the host. The host may be any of cocci, spirilla, and bacilli, and bacilli are preferred.

Examples of the prokaryotic host such as bacteria can include microorganisms belonging to the genera *Escherichia, Brevibacillus*, *Serratia, Bacillus, Microbacterium, Brevibacterium*, *Corynebacterium,* and *Pseudomonas.* Preferable examples of the prokaryote can include *Escherichia coli, Bacillus subtilis, Pseudomonas, Corynebacterium, and Lactococcus.* The host cells are preferably microorganisms belonging to the genus *Escherichia,* particularly *Escherichia coli.*

Examples of the microorganisms belonging to the genus *Escherichia* can include *Escherichia coli* BL21 (Novagen, Inc.), *Escherichia coli* BL21(DE3) (Life Technologies Corporation), *Escherichia coli* BLR(DE3) (Merck Millipore), *Escherichia coli* DH1, *Escherichia coli* GI698, *Escherichia coli* HB101, *Escherichia coli* JM109, *Escherichia coli* K5 (ATCC 23506), *Escherichia coli* KY3276, *Escherichia coli* MC1000, *Escherichia coli* MG1655 (ATCC 47076), *Escherichia coli* No.49, *Escherichia coli* Rosetta(DE3) (Novagen, Inc.), *Escherichia coli* TB1, *Escherichia coli* Tuner (Novagen, Inc.), *Escherichia coli* Tuner(DE3) (Novagen, Inc.), *Escherichia coli* W1485, *Escherichia coli* W3110 (ATCC 27325), *Escherichia coli* XL1-Blue, and *Escherichia coli* XL2-Blue. The host cells are preferably *Escherichia coli.*

Examples of the eukaryotic host include yeasts, and filamentous fungi (molds and the like).

Examples of the yeast can include yeasts belonging to the genera Saccharomyces, Schizosaccharomyces, Kluyveromyces, Trichosporon, Schwanniomyces, Pichia, Candida, Yarrowia, and Hansenula.

Examples of the filamentous fungus can include fungi belonging to the genera Acremonium, Aspergillus, Ustilago, Trichoderma, Neurospora, Fusarium, Humicola, Penicillium, Myceliophtora, Botryts, Magnaporthe, Mucor, Metarhizium, Monascus, Rhizopus, and Rhizomucor.

### (First step)

In the first step, recombinant cells expressing a recombinant protein under the control of an inducible promoter are cultured in a medium containing galactose and at least two selected from the group consisting of mannitol, raffinose, and melibiose.

The medium may contain at least two of mannitol, raffinose, and melibiose in addition to galactose. The medium may be, for example, a protein production medium to which galactose and at least two selected from the group consisting of mannitol, raffinose, and melibiose are added.

The protein production medium is not particularly limited, and can be selected from known natural medium or synthetic medium depending on the type of the recombinant cells. As the protein production medium, for example, a liquid medium containing components selected from a carbon source, a nitrogen source, a phosphoric acid source, a sulfur source, vitamins, a mineral, a nutrient required by auxotrophy, and other various organic components and inorganic components as necessary can be used. The type and concentration of the medium component may be set as appropriate by those skilled in the art.

Examples of the carbon source can include saccharides such as glucose, sucrose, lactose, and fructose, and a hydrolyzate of starch, alcohols such as glycerol and sorbitol, and organic acids such as fumaric acid, citric acid, and succinic acid.

One type of the carbon source may be contained in the medium, or a mixture of two or more types of the carbon sources may be contained at an optional ratio. The concentration of the carbon source in the protein production medium may be approximately 0.1 w/v% to 50 w/v%, preferably approximately 0.5 w/v% to 40 w/v%, more preferably approximately 1 w/v% to 30 w/v%, and particularly preferably approximately 5 w/v% to 20 w/v%. In the present embodiment, it is preferable to use glycerol or glucose as the carbon source, and glycerol or glucose may be mixed with another carbon source at an optional ratio. The proportion of glycerol or glucose in the carbon source is preferably 10 wt% or more, more preferably 50 wt% or more, and particularly preferably 70 wt% or more. The preferred initial concentration of the carbon source at the start of the culture is as described above, and the carbon source may be added as appropriate depending on consumption of the carbon source during the culture.

Examples of the nitrogen source include inorganic nitrogen salts such as nitrate, ammonium salt, ammonia gas, and aqueous ammonia, and organic nitrogen sources such as amino acids, peptones, and extracts. Examples of the peptone can include casein peptone, meat peptone, heart muscle peptone, gelatin peptone, and soy peptone. Examples of the extract can include meat extract, yeast extract, and heart infusion. In the nitrogen source containing an amino acid or a peptide, it is preferable that contents of the lower molecular weight peptide and the amino acid are large.

Examples of the phosphoric acid source can include phosphates such as potassium dihydrogen phosphate and dipotassium hydrogen phosphate and phosphoric acid polymers such as pyrophosphoric acid.

Examples of the sulfur source can include inorganic sulfur compounds such as sulfate, thiosulfate, and sulfite and sulfur-containing amino acids such as cysteine, cystine, and glutathione.

Examples of the vitamin can include biotin, choline chloride, cyanocobalamin, folic acid, inositol, nicotinic acid, 4-aminobenzoic acid, pantothenic acid, pyridoxine, riboflavin, thiamine, and thymidine. Examples of the source of the vitamin can include various extracts such as malt extract, potato extract, and tomato juice.

Examples of the mineral can include sulfur (S), potassium (K), calcium (Ca), magnesium (Mg), iron (Fe), sodium (Na), and the like, in addition to phosphorus (P).

The content of galactose in the medium may be 0.1 mg to 300 mg, 0.5 mg to 250 mg, 1 mg to 200 mg, 1 mg to 150 mg, 1 mg to 100 mg, 5 mg to 200 mg, 5 mg to 150 mg, 10 mg to 200 mg, 10 mg to 150 mg, 10 mg to 100 mg, 10 mg to 80 mg, 10 mg to 50 mg, 15 to 150 mg, 20 mg to 150 mg, 20 mg to 100 mg, 25 mg to 125 mg, 50 mg to 150 mg, 50 mg to 120 mg, and 50 mg to 100 mg, per 500 mL of the medium. When the content of galactose is in this range, the production amount of the recombinant protein is further increased.

When the medium contains mannitol, the concentration of mannitol in the medium may be 0.1 mg to 300 mg, 0.5 mg to 250 mg, 1 mg to 200 mg, 1 mg to 150 mg, 1 mg to 100 mg, 5 mg to 200 mg, 5 mg to 150 mg, 10 mg to 200 mg, 10 mg to 150 mg, 10 mg to 100 mg, 10 mg to 80 mg, 10 mg to 50 mg, 15 to 150 mg, 20 mg to 150 mg, 20 mg to 100 mg, 25 mg to 125 mg, 50 mg to 150 mg, 50 mg to 120 mg, and 50 mg to 100 mg, per 500 mL of the medium. When the content of mannitol is in this range, the production amount of the recombinant protein is further increased.

When the medium contains raffinose, the concentration of raffinose in the medium may be 0.1 mg to 300 mg, 0.5 mg to 250 mg, 1 mg to 200 mg, 1 mg to 150 mg, 1 mg to 100 mg, 5 mg to 200 mg, 5 mg to 150 mg, 10 mg to 200 mg, 10 mg to 150 mg, 10 mg to 100 mg, 10 mg to 80 mg, 10 mg to 50 mg, 15 to 150 mg, 20 mg to 150 mg, 20 mg to 100 mg, 25 mg to 125 mg, 50 mg to 150 mg, 50 mg to 120 mg, and 50 mg to 100 mg, per 500 mL of the medium. When the content of raffinose is in this range, the production amount of the recombinant protein is further increased.

When the medium contains melibiose, the concentration of melibiose in the medium may be 0.1 mg to 300 mg, 0.5 mg to 250 mg, 1 mg to 200 mg, 1 mg to 150 mg, 1 mg to 100 mg, 5 mg to 200 mg, 5 mg to 150 mg, 10 mg to 200 mg, 10 mg to 150 mg, 10 mg to 100 mg, 10 mg to 80 mg, 10 mg to 50 mg, 15 to 150 mg, 20 mg to 150 mg, 20 mg to 100 mg, 25 mg to 125 mg, 50 mg to 150 mg, 50 mg to 120 mg, and 50 mg to 100 mg, per 500 mL of the medium. When the content of melibiose is in this range, the production amount of the recombinant protein is further increased.

When the medium contains galactose and at least two selected from the group consisting of mannitol, raffinose, and melibiose, the total concentration of galactose and at least two selected from the group consisting of mannitol, raffinose, and melibiose in the medium may be 0.2 mg to 600 mg, 0.2 mg to 400 mg, 1 mg to 600 mg, 1 mg to 400 mg, 1 mg to 300 mg, 1 mg to 200 mg, 1 mg to 100 mg, 10 mg to 600 mg, 10 mg to 400 mg, 10 mg to 300 mg, 10 mg to 200 mg, 10 mg to 100 mg, 20 mg to 600 mg, 20 mg to 400 mg, 20 mg to 300 mg, 20 mg to 200 mg, 20 mg to 100 mg, 50 mg to 600 mg, 50 mg to 400 mg, 50 mg to 300 mg, 50 to 200 mg, 100 mg to 600 mg, 100 mg to 400 mg, 100 mg to 300 mg, and 100 mg to 200 mg, per 500 mL of the medium. When the total content of galactose and at least two selected from the group consisting of mannitol, raffinose, and melibiose is in this range, the production amount of the recombinant protein is further increased.

When the medium contains galactose and at least three selected from the group consisting of mannitol, raffinose, and melibiose, the total concentration of galactose and at least three selected from the group consisting of mannitol, raffinose, and melibiose in the medium may be 1 mg to 900 mg, 1 mg to 600 mg, 10 mg to 600 mg, 15 mg to 600 mg, 15 mg to 500 mg, 15 mg to 400 mg, 15 mg to 300 mg, 20 mg to 600 mg, 20 mg to 500 mg, 20 mg to 400 mg, 20 mg to 300 mg, 30 mg to 600 mg, 30 mg to 500 mg, 30 mg to 400 mg, 30 mg to 300 mg, 45 mg to 600 mg, 45 mg to 500 mg, 45 mg to 400 mg, 45 mg to 300 mg, 60 mg to 600 mg, 60 mg to 500 mg, 60 mg to 400 mg, 60 mg to 300 mg, 90 mg to 900 mg, 90 mg to 600 mg, 90 mg to 500 mg, 90 mg to 400 mg, 90 mg to 300 mg, 120 mg to 900 mg, 120 mg to 600 mg, 120 mg to 500 mg, 120 mg to 400 mg, 120 mg to 300 mg, 150 mg to 900 mg, 150 mg to 600 mg, 150 mg to 500 mg, 150 mg to 400 mg, and 150 mg to 300 mg, per 500 mL of the medium. When the total content of galactose and at least three selected from the group consisting of mannitol, raffinose, and melibiose is in this range, the production amount of the recombinant protein is further increased.

The medium may contain all of galactose, mannitol, raffinose, and melibiose. When the medium contains all of galactose, mannitol, raffinose, and melibiose, the total concentration of galactose, mannitol, raffinose, and melibiose in the medium may be 0.5 mg or more, 5 mg or more, 10 mg or more, 30 mg or more, 40 mg or more, 50 mg or more, 60 mg or more, 80 mg or more, 100 mg or more, 120 mg or more, 150 mg or more, 200 mg or more, 300 mg or more, 400 mg or more, 800 mg or less, 600 mg or less, 500 mg or less, 400 mg or less, 40 mg to 800 mg, 40 mg to 600 mg, 40 mg to 400 mg, 200 mg to 800 mg, 200 mg to 600 mg, and 200 mg to 400 mg, per 500 mL of the medium. As a result, the production amount of the recombinant protein is further increased. The medium may also contain galactose, mannitol, and raffinose. As a result, the production amount of the recombinant protein is further increased, and the production cost can be further reduced.

The culture in the first step can be performed aerobically by, for example, aeration culture or shaking culture. The culture can be performed by batch culture, fed-batch culture, continuous culture, or a combination thereof. The pH of the medium may be, for example, 3.0 to 9.0. The culture temperature may be, for example, 15 to 40°C. The culture time may be, for example, 1 to 60 hours.

In the production method according to the present embodiment, a recombinant protein is expressed by the culture in the first step. The production amount of the recombinant protein in the first step is usually lower than the production amount (normal production amount) of the recombinant protein when recombinant cells are cultured under the condition that expression from the inducible promoter is induced without performing the first step. On the other hand, by combining the first step and the second step described below, a production amount higher than the normal production amount can be achieved.

### (Second step)

The second step is a step of further culturing the recombinant cells after the first step under the condition that expression from the inducible promoter is induced. By subjecting the recombinant cells to the condition that expression from the inducible promoter is induced, transcription by the inducible promoter (transcription of the nucleic acid encoding the recombinant protein) is activated, and thus expression of the recombinant protein is further induced. The "condition that expression from the inducible promoter is induced" in the second step means that the medium contains galactose and at least two selected from the group consisting of mannitol, raffinose, and melibiose, and in addition, the condition that expression from an inducible promoter other than these is induced is further set.

The activation of transcription by the inducible promoter can be performed according to a method known in the technical field, depending on the type of the inducible promoter. For example, when an inducible promoter (for example, an IPTG inducible promoter) activated by the presence of an inducer (expression inducer) is used, transcription by the inducible promoter is activated by adding the inducer (for example, IPTG) to a culture solution, and thus expression of the recombinant protein can be induced. The inducer may be added to the culture solution all at once or in several portions, or it may be added to the culture solution as a continuous feed. Feeding may also be performed by addition of the inducer to the feed substrate solution. The amount of the inducer added can be set according to the type of the inducer and the inducible promoter. The amount can be, for example, in the range of 0.1 to 30 µg and preferably in the range of 0.5 to 20 µg, per 1 g of a dry weight of the recombinant cells.

When an inducible promoter activated by increase or decrease in the temperature is used, for example, transcription by the inducible promoter is activated by increasing or decreasing the temperature of the culture solution, and thus expression of the recombinant protein can be induced.

There is no particular restriction on the period from the first step to the second step, and it can be appropriately set according to the culture system configuration and the production process design. From the viewpoint of efficient production of the recombinant protein, it is preferred to move to the second step when the growth of the recombinant cells has reached the metaphase to the anaphase of the logarithmic growth stage in the first step.

The growth of the recombinant cells begins from the lag phase or induction phase (the period of delayed increase in the initial cell count), and through the logarithmic growth stage (the period of logarithmic increase to twice the cell count per unit time), reaches the stationary phase (the period where no net change is seen in the number of cells). The metaphase of the logarithmic growth stage is the period in which the cell count is midway between the cell count in the lag phase and the cell count in the stationary phase, and the anaphase of the logarithmic growth stage is the period from the metaphase until the stationary phase. As a specific example of the period of moving from the first step to the second step, for recombinant cells in which the OD₆₀₀ value at the stationary phase is approximately 150, it is preferably the period in which the OD₆₀₀ value has reached 30 to 120, more preferably the period in which it has reached 40 to 110, and even more preferably the period in which it has reached 60 to 100.

The culture time in the second step may be a time length until the predetermined production amount has been obtained, which will depend on the type of host used and recombinant protein. Since the production rate varies depending on the culture conditions such as the temperature of the culture solution, it is not necessary to absolutely specify the culture time in the second step. The culture time in the second step may also be set to match progression to separation and purification of the recombinant protein in the subsequent step. For industrial production, it is preferred to set the time for the induction of the recombinant protein expression so as not to affect the growth of the recombinant cells being carried out in parallel, or transfer of the grown recombinant cells. Examples of the culture time in the second step include, but are not limited to, 1 hour to 30 hours.

The culture solution used for expression of the recombinant protein is induced in the second step may be used for separation and purification of the following recombinant protein.

### (Separation and purification of recombinant protein)

Separation and purification of the recombinant protein can be performed by a commonly used method. For example, in a case where the recombinant protein is expressed in a dissolved state in cells, the host (recombinant cells) is collected by centrifugation after completion of the culture in the second step, the collected cells are suspended in an aqueous buffer, and then the recombinant cells are disrupted using an ultrasonicator, a French press, a Manton-Gaulin homogenizer, a Dyno-Mill, or the like to obtain a cell-free extract. A purified preparation of the recombinant protein can be obtained from the supernatant obtained by centrifuging the cell-free extract, according to a method commonly used for protein isolation and purification, that is, a solvent extraction method, a salting-out method using ammonium sulfate or the like, a desalting method, a precipitation method using an organic solvent, an anion exchange chromatography method using a resin such as diethylaminoethyl (DEAE)-sepharose or DIAION HPA-75 (manufactured by Mitsubishi Kasei Kogyo Kabushiki Kaisha), a cation exchange chromatography method using a resin such as S-sepharose FF (manufactured by Pharmacia Corporation), a hydrophobic chromatography method using a resin such as butyl sepharose and phenyl sepharose, a gel filtration method using a molecular sieve, an affinity chromatography method, a chromatofocusing method, an electrophoresis method such as isoelectric focusing phoresis and the like, alone or in combination thereof.

In a case where the recombinant protein is expressed with formation of an insoluble matter in cells, similarly, the host (recombinant cells) is collected, disrupted, and centrifuged to collect the insoluble matter of the recombinant protein as a precipitated fraction. The collected insoluble matter of the recombinant protein can be solubilized with a protein modifier. After this operation, a purified preparation of the recombinant protein can be obtained by the same isolation and purification method as described above.

In a case where the recombinant protein is secreted extracellularly, the recombinant protein can be collected from a culture supernatant. That is, the culture supernatant is obtained by treating a culture solution by a technique such as centrifugation, and a purified preparation of a recombinant protein can be obtained from the culture supernatant by using the same isolation and purification method as described above.

### Examples

Hereinafter, the present invention will be described more specifically based on Examples and the like. However, the present invention is not limited to the following Examples.

### [Production Example 1: production of modified fibroin by chromosome-integrated expression strain]

### (1-1) Production of modified fibroin expression strain (chromosome-integrated expression strain)

Modified fibroin (hereinafter, also referred to as "PRT410") having the amino acid sequence listed as SEQ ID NO: 1 was designed based on the nucleotide sequence and amino acid sequence of fibroin derived from *Nephila clavipes* (GenBank Accession No.: P46804.1, GI: 1174415).

The amino acid sequence listed as SEQ ID NO: 2 (Met-PRT410) is obtained by substituting, inserting, and deleting amino acid residues in the amino acid sequence of fibroin derived from *Nephila clavipes* for the purpose of improving productivity. The amino acid sequence listed as SEQ ID NO: 1 (PRT410) is obtained by adding the amino acid sequence listed as SEQ ID NO: 3 (tag sequence and hinge sequence) to the N-terminal of the amino acid sequence listed as SEQ ID NO: 2.

Then, nucleic acid encoding PRT410 was synthesized. The nucleic acid had an NdeI site added at the 5' end and an EcoRI site added downstream from the stop codon. The nucleic acid was cloned in a cloning vector (pUC118). The nucleic acid was then subjected to restriction enzyme treatment with NdeI and EcoRI for cleavage, after which it was recombined with a pET-22b(+) vector to obtain a pET-22(+)/PRT410 vector.

A modified fibroin (PRT410) expression cassette was integrated into the host chromosome using a homologous recombination system of λ phage. In the homologous recombination system, homologous recombination occurs by the products of the exo, bet, and gam genes which are in the Red region in the phage genome.

First, a modified fibroin (PRT410) expression cassette (including a manX5' homologous sequence, a T7 promoter, PRT410, and a T7 terminator in this order) was amplified by a PCR method using a pET-22(+)/PRT410 vector as a template. Similarly, a kanamycin resistance gene expression cassette (including a T7 terminator homologous sequence, FRT, kanamycin resistance gene, FRT, and a manX3' homologous sequence in this order) was amplified by a PCR method using a pKD13-Km vector as a template. The two PCR products were joined using the In-Fusion (registered trademark) cloning system (manufactured by Takara Bio Inc.). Next, the joined DNA fragment was introduced into a host (*Escherichia coli* BL21 (DE3) strain) to integrate the modified fibroin (PRT410) expression cassette into the host chromosome by homologous recombination between the manX5' homologous sequence on the host chromosome and the manX5' homologous sequence on the DNA fragment and between the manX3' homologous sequence on the host chromosome and the manX3' homologous sequence on the DNA fragment. Note that the exo, bet, and gam genes had been expressed in the host in advance by introducing the helper plasmid pKD46 having these genes (Proc. Natl. Acad. Sci. USA, 97: 6640-6645) .

The transformed strain and *Escherichia coli* BLR (DE3) were mixed at a ratio of 1 : 1, and the mixture was cultured in a plate medium containing LB and kanamycin. A strain in which the nucleic acid has been integrated into the chromosome was selected from strains exhibiting kanamycin resistance and ampicillin sensitivity. Thereafter, a helper plasmid pCP20 was introduced into the selected strain to express FLP, thereby removing kanamycin resistance gene sandwiched between FRT sequences. Whereby, a modified fibroin (PRT410) expression strain (chromosome-integrated expression strain) was obtained.

### (1-2) Seed culture

The chromosome-integrated expression strain produced in the above (1-1) was cultured in 2 mL of LB medium for 15 hours. The culture solution was added to 100 mL of medium for seed culture (Table 1) so that the OD₆₀₀ was 0.005, the culture solution temperature was maintained at 30°C, and flask culture was performed until the OD₆₀₀ reached 5 (approximately 15 hours) to obtain a seed culture solution.

**[Table 1]**

| Medium for seed culture | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |

### (1-3) Preparation of medium for main culture

In a culture tank, 500 mL of medium for main culture (Table 2) and predetermined amounts of galactose, mannitol, raffinose, and melibiose were added, and sterilized in an autoclave (TOMY LSX-500) at 121°C for 20 minutes. After cooling to 37°C, the pH of the medium was adjusted to 6.1 to 6.3 using 28 to 30% aqueous ammonia (Kanto Chemical Co., Inc. 01266-88).

**[Table 2]**

| Medium for main culture | |
|---|---|
| Reagent | Concentration (g/L) |
| Glucose | 10.0 |
| KH₂PO₄ | 22.0 |
| FeSO₄·7H₂O | 0.04 |
| CaCl₂·2H₂O | 0.04 |
| MgSO₄·7H₂O | 2.4 |
| Yeast Extract | 11.25 |
| Antifoaming agent (ADEKA, LG-295S) | 1.0 (mL/L) |

### (1-4) Preparation of feed substrate solution

A predetermined amount of feed substrate solution (Table 3) was added to a feed pot, and sterilized in an autoclave (TOMY LSX-500) at 121°C for 20 minutes.

**[Table 3]**

| Feed substrate solution | |
|---|---|
| Reagent | Concentration (g/L) |
| Glucose | 600.0 |
| MgSO₄·7H₂O | 10.0 |

### (1-5) Culture and induction of expression

### <First step>

The medium for main culture (initial amount of medium: 0.5 L) prepared in (1-3) was loaded into a culture tank, and the seed culture solution obtained in (1-2) was added so that the OD₆₀₀ was 0.05. The temperature of the culture solution was maintained at 37°C, and main culture was performed while the pH was controlled to be constant at 6.9 with 30% aqueous ammonia and a 4 M phosphoric acid solution (Wako Pure Chemical Industries, Ltd.). In addition, the mixture was stirred by aeration so that the dissolved oxygen concentration in the culture solution was maintained at 30 to 40% of the dissolved oxygen saturation concentration. Immediately after glucose in the production medium was completely consumed, the feed substrate solution prepared in (1-3) was fed at a constant rate of 6 g/h.

### <Second step>

The fed-batch culture was continued until the OD₆₀₀ of the culture solution reached approximately 60, then IPTG (expression inducer) was added to the culture tank so as to be 0.1 mM, and induction of expression of the modified fibroin (PRT410) was started. Culture was performed until the lapse of 24 hours after the addition of IPTG.

The culture solution was centrifuged at the time of addition of IPTG (when the first step was completed, and also when the second step started) or 24 hours after the addition of IPTG (when the second step was completed), and bacterial cells were collected. The collected bacterial cells were washed with a 20 mM Tris-HCl buffer (pH 7.4). The washed bacterial cells were suspended in a 20 mM Tris-HCl buffer solution (pH 7.4) containing approximately 1 mM PMSF, and the cells were disrupted with a high-pressure homogenizer (manufactured by GEA Niro Soavi). The disrupted cells were centrifuged to obtain a precipitate. The obtained precipitate was washed with a 20 mM Tris-HCl buffer (pH 7.4) until the purity of the precipitate became high. The precipitate after washing was suspended in an 8 M guanidine buffer (8 M guanidine hydrochloride, 10 mM sodium dihydrogen phosphate, 20 mM NaCl, 1 mM Tris-HCl, pH 7.0) so that the concentration thereof was 100 mg/mL, and dissolved by stirring with a stirrer for 30 minutes at 60°C. After dissolution, dialysis was performed with water using a dialysis tube (cellulose tube 36/32, manufactured by Sanko Junyaku Co., Ltd.). A white aggregated protein obtained after the dialysis was collected by centrifugation, moisture was removed with a lyophilizer, and the lyophilized powder was collected, thereby obtaining the modified fibroin (PRT410).

The production amount of the modified fibroin was evaluated by conducting polyacrylamide gel electrophoresis on the obtained lyophilized powder and conducting image analysis using Totallab (Nonlinear Dynamics Ltd.). The production amount of each modified fibroin calculated from the weight of the lyophilized powder was calculated as a relative value when the production amount (control) when cells were cultured in a medium for main culture to which galactose, mannitol, raffinose, and melibiose were not added was taken as 100%.

### (1-6) Results

Results are shown in Tables 4 and 5.

**[Table 4]**

| | Additive component (mg/500 mL culture solution) | | | | 24 At time of IPTG addition | hours after IPTG addition |
|---|---|---|---|---|---|---|
| | Mannitol | Raffinose | Galactose | Melibiose | | |
| Control | 0 | 0 | 0 | 0 | 11% | 100% |
| Example 1 | 50 | 50 | 50 | 50 | 48% | 121% |
| Example 2 | 100 | 100 | 100 | 100 | 67% | 132% |
| Example 3 | 150 | 150 | 150 | 150 | 7 3% | 112% |
| Example 4 | 200 | 200 | 200 | 200 | 78% | 106% |

As shown in Table 4, by adding galactose, mannitol, raffinose, and melibiose, the production amount of modified fibroin at the time of addition of IPTG (at the time of transition from the first step to the second step) increased depending on the addition concentration. In addition, by adding IPTG to further induce expression, the final production amount of the modified fibroin increased as compared with the case where galactose, mannitol, raffinose, and melibiose were not added (control) (Examples 1 to 4, 24 hours after the addition of IPTG).

**[Table 5]**

| | Additive component (mg/500 mL culture solution) | | | | 24 hours after IPTG addition |
|---|---|---|---|---|---|
| | Mannitol | Raffinose Galactose | | Melibiose | |
| Control | 0 | 0 | 0 | 0 | 100% |
| Example 5 | 100 | 100 | 100 | 100 | 129% |
| Example 6 | 100 | 100 | 100 | 0 | 109% |
| Example 7 | 0 | 100 | 100 | 100 | 120% |
| Example 8 | 100 | 0 | 100 | 100 | 123% |

As shown in Table 5, by adding galactose and further adding two selected from mannitol, raffinose, and melibiose, the final production amount of the modified fibroin increased as compared with the case where galactose, mannitol, raffinose, and melibiose were not added (control) (Examples 6 to 8, 24 hours after the addition of IPTG).

### [Production Example 2: production of modified fibroin by plasmid-type expression strain]

### (2-1) Production of modified fibroin expression strain (plasmid-type expression strain)

Modified fibroin (hereinafter, also referred to as "PRT918") having the amino acid sequence listed as SEQ ID NO: 4 was designed based on the nucleotide sequence and amino acid sequence of fibroin derived from *Nephila clavipes* (GenBank Accession No.: P46804.1, GI: 1174415).

The amino acid sequence listed as SEQ ID NO: 5 (Met-PRT918) is obtained by substituting, inserting, and deleting amino acid residues in the amino acid sequence of fibroin derived from *Nephila clavipes* for the purpose of improving productivity. In the amino acid sequence listed as SEQ ID NO: 4 (PRT966), the amino acid sequence listed as SEQ ID NO: 3 (tag sequence and hinge sequence) is added at the N-terminal of the amino acid sequence listed as SEQ ID NO: 5.

Then, nucleic acid encoding PRT918 was synthesized. The nucleic acid had an NdeI site added at the 5' end and an EcoRI site added downstream from the stop codon. The nucleic acid was cloned in a cloning vector (pUC118). The nucleic acid was then subjected to restriction enzyme treatment with NdeI and EcoRI for cleavage, after which it was recombined with a pET-22b(+) vector to obtain a pET-22(+)/PRT918 vector.

*Escherichia coli* BLR (DE3) was transformed with the obtained pET-22(+)/PRT918 vector to obtain a modified fibroin (PRT918) expression strain (plasmid-type expression strain). In this vector, the modified fibroin (PRT918) is expressed under the control of the T7 promoter.

### (2-2) Seed culture

The plasmid-type expression strain produced in the above (2-1) was cultured in 2 mL of LB medium containing ampicillin for 15 hours. The culture solution was added to 100 mL of medium for seed culture (Table 6) so that the OD₆₀₀ was 0.005, the culture solution temperature was maintained at 30°C, and flask culture was performed until the 0D₆₀₀ reached 5 (approximately 15 hours) to obtain a seed culture solution.

**[Table 6]**

| Medium for seed culture | |
|---|---|
| Reagent | Concentration (g/L) |
| Glucose | 5.0 |
| KH₂PO₄ | 4.0 |
| K₂HPO₄ | 9.3 |
| Yeast Extract | 6.0 |
| Ampicillin | 0.1 |

### (2-3) Preparation of medium for main culture

In a culture tank, 1,500 mL of medium for main culture (Table 7) and predetermined amounts of galactose, mannitol, raffinose, and melibiose were added, and sterilized in an autoclave (TOMY LSX-500) at 121°C for 20 minutes. After cooling to 37°C, the pH of the medium was adjusted to 6.1 to 6.3 using 28 to 30% aqueous ammonia (Kanto Chemical Co., Inc. 01266-88).

**[Table 7]**

| Medium for main culture | |
|---|---|
| Reagent | Concentration (g/L) |
| Glucose | 10.0 |
| KH₂PO₄ | 22.0 |
| FeSO₄·7H₂O | 0.04 |
| CaCl₂·2H₂O | 0.04 |
| MgSO₄·7H₂O | 2.4 |
| Yeast Extract | 11.25 |
| Antifoaming agent (ADEKA, LG-295S) | 1.0 (mL/L) |

### (2-4) Preparation of feed substrate solution

A feed substrate solution was prepared in the same manner as in (1-4).

### (2-5) Culture and induction of expression

### <First step>

The medium for main culture (initial amount of medium: 1.5 L) prepared in (2-3) was loaded into a culture tank, and the seed culture solution obtained in (2-2) was added so that the OD₆₀₀ was 0.05. The temperature of the culture solution was maintained at 37°C, and main culture was performed while the pH was controlled to be constant at 6.9 with 30% aqueous ammonia and a 4 M phosphoric acid solution (Wako Pure Chemical Industries, Ltd.). In addition, the mixture was stirred by aeration so that the dissolved oxygen concentration in the culture solution was maintained at 30 to 40% of the dissolved oxygen saturation concentration. Immediately after glucose in the production medium was completely consumed, the feed substrate solution prepared in (2-4) was fed at a constant rate of 6 g/h.

### <Second step>

The fed-batch culture was continued until the OD₆₀₀ of the culture solution reached approximately 100, then IPTG (expression inducer) was added to the culture tank so as to be 0.1 mM, and induction of expression of the modified fibroin (PRT918) was started. Culture was performed until the lapse of 10 hours after the addition of IPTG.

Ten hours after the addition of IPTG (when the second step was completed), the culture solution was centrifuged, and bacterial cells were collected. A lyophilized powder of the modified fibroin (PRT918) was obtained from the collected bacterial cells in the same procedure as in (1-5), and the production amount of the modified fibroin was evaluated. The production amount of each modified fibroin calculated from the weight of the lyophilized powder was calculated as a relative value when the production amount (control) when cells were cultured in a medium for main culture to which galactose, mannitol, and raffinose were not added was taken as 100%.

### (2-6) Results

Results are shown in Table 8.

**[Table 8]**

| | Additive component (mg/500 mL culture solution) | | | 10 hours after IPTG addition |
|---|---|---|---|---|
| | Mannitol | Raffinose | Galactose | |
| Control | 0 | 0 | 0 | 100% |
| Example 9 | 1 | 1 | 1 | 107% |
| Example 10 | 10 | 10 | 10 | 102% |
| Example 11 | 20 | 20 | 20 | 123% |
| Example 12 | 50 | 50 | 50 | 139% |
| Example 13 | 100 | 100 | 100 | 135% |

As shown in Table 8, by adding galactose, mannitol, and raffinose, the final production amount of the modified fibroin increased as compared with the case where galactose, mannitol, and raffinose were not added (control) (Examples 9 to 13, 10 hours after the addition of IPTG).

## Claims

1. A method for producing a recombinant protein, the method comprising:
a first step of culturing a recombinant cell expressing a recombinant protein under control of an inducible promoter in a medium containing galactose and at least two selected from the group consisting of mannitol, raffinose, and melibiose; and
a second step of further culturing the recombinant cell under a condition that expression from the inducible promoter is induced after the first step.

2. The production method according to claim 1, wherein the inducible promoter is an isopropyl-β-thiogalactopyranoside (IPTG) inducible promoter.

3. The production method according to claim 1 or 2, wherein the inducible promoter is a T7 promoter.

4. The production method according to claim 2 or 3, wherein the second step is a step of adding IPTG to the medium and further culturing the recombinant cell.

5. The production method according to any one of claims 1 to 4, wherein the medium contains galactose, mannitol, raffinose, and melibiose.

6. The production method according to any one of claims 1 to 4, wherein the medium contains galactose, mannitol, and raffinose.

7. The production method according to any one of claims 1 to 6, wherein the recombinant protein is a structural protein.

8. The production method according to any one of claims 1 to 7, wherein the recombinant protein is fibroin.

9. The production method according to any one of claims 1 to 8, wherein the recombinant protein is spider silk fibroin.

10. The production method according to any one of claims 1 to 9, wherein the recombinant cell is a bacillus.

11. The production method according to any one of claims 1 to 10, wherein the recombinant cell is a microorganism belonging to the genus *Escherichia.*


SEQUENCE LISTING

<110>  Spiber Inc.

<120>  A method of producing recombinant protein

<130>  FP20-0294-00

<150>  JP2019-070585
<151>  2019-04-02

<160>  5

<170>  PatentIn version 3.5

<210>  1
<211>  601
<212>  PRT
<213>  Artificial Sequence

<220>
<223>  PRT410

<400>  1

Met His His His His His His Ser Ser Gly Ser Ser Gly Pro Gly Gln
1               5                   10                  15

Gln Gly Pro Tyr Gly Pro Gly Ala Ser Ala Ala Ala Ala Ala Gly Gln
            20                  25                  30

Asn Gly Pro Gly Ser Gly Gln Gln Gly Pro Gly Gln Ser Gly Gln Tyr
        35                  40                  45

Gly Pro Gly Gln Gln Gly Pro Gly Gln Gln Gly Pro Gly Ser Ser Ala
    50                  55                  60

Ala Ala Ala Ala Gly Pro Gly Gln Tyr Gly Pro Gly Gln Gln Gly Pro
65                  70                  75                  80

Ser Ala Ser Ala Ala Ala Ala Ala Gly Pro Gly Ser Gly Gln Gln Gly
                85                  90                  95

Pro Gly Ala Ser Gly Gln Tyr Gly Pro Gly Gln Gln Gly Pro Gly Gln
            100                 105                 110

Gln Gly Pro Gly Ser Ser Ala Ala Ala Ala Ala Gly Gln Tyr Gly Ser
        115                 120                 125

Gly Pro Gly Gln Gln Gly Pro Tyr Gly Ser Ala Ala Ala Ala Ala Gly
    130                 135                 140

Pro Gly Ser Gly Gln Tyr Gly Gln Gly Pro Tyr Gly Pro Gly Ala Ser
145                 150                 155                 160

Gly Pro Gly Gln Tyr Gly Pro Gly Gln Gln Gly Pro Ser Ala Ser Ala
                165                 170                 175

Ala Ala Ala Ala Gly Ser Gly Gln Gln Gly Pro Gly Gln Tyr Gly Pro
            180                 185                 190

Tyr Ala Ser Ala Ala Ala Ala Ala Gly Gln Tyr Gly Ser Gly Pro Gly
        195                 200                 205

Gln Gln Gly Pro Tyr Gly Pro Gly Gln Ser Gly Ser Gly Gln Gln Gly
    210                 215                 220

Pro Gly Gln Gln Gly Pro Tyr Ala Ser Ala Ala Ala Ala Ala Gly Pro
225                 230                 235                 240

Gly Gln Gln Gly Pro Tyr Gly Pro Gly Ser Ser Ala Ala Ala Ala Ala
                245                 250                 255

Gly Gln Tyr Gly Tyr Gly Pro Gly Gln Gln Gly Pro Tyr Gly Pro Gly
            260                 265                 270

Ala Ser Gly Gln Asn Gly Pro Gly Ser Gly Gln Tyr Gly Pro Gly Gln
        275                 280                 285

Gln Gly Pro Gly Gln Ser Ala Ala Ala Ala Ala Gly Pro Gly Gln Gln
    290                 295                 300

Gly Pro Tyr Gly Pro Gly Ala Ser Ala Ala Ala Ala Ala Gly Gln Tyr
305                 310                 315                 320

Gly Pro Gly Gln Gln Gly Pro Gly Gln Tyr Gly Pro Gly Ser Ser Gly
                325                 330                 335

Pro Gly Gln Gln Gly Pro Tyr Gly Pro Gly Ser Ser Ala Ala Ala Ala
            340                 345                 350

Ala Gly Gln Tyr Gly Pro Gly Gln Gln Gly Pro Tyr Gly Pro Gly Gln
        355                 360                 365

Ser Ala Ala Ala Ala Ala Gly Gln Tyr Gln Gln Gly Pro Gly Gln Gln
    370                 375                 380

Gly Pro Tyr Gly Pro Gly Ala Ser Gly Pro Gly Gln Gln Gly Pro Tyr
385                 390                 395                 400

Gly Pro Gly Ala Ser Ala Ala Ala Ala Ala Gly Pro Gly Gln Tyr Gly

405                 410                 415

Pro Gly Gln Gln Gly Pro Ser Ala Ser Ala Ala Ala Ala Ala Gly Gln
            420                 425                 430

Tyr Gly Ser Gly Pro Gly Gln Tyr Gly Pro Tyr Gly Pro Gly Gln Ser
        435                 440                 445

Gly Pro Gly Ser Gly Gln Gln Gly Gln Gly Pro Tyr Gly Pro Gly Ala
    450                 455                 460

Ser Ala Ala Ala Ala Ala Gly Gln Tyr Gly Pro Gly Gln Gln Gly Pro
465                 470                 475                 480

Tyr Gly Pro Gly Gln Ser Ala Ala Ala Ala Ala Gly Pro Gly Ser Gly
                485                 490                 495

Gln Tyr Gly Pro Gly Ala Ser Gly Gln Asn Gly Pro Gly Ser Gly Gln
            500                 505                 510

Tyr Gly Pro Gly Gln Gln Gly Pro Gly Gln Ser Ala Ala Ala Ala Ala
        515                 520                 525

Gly Gln Tyr Gln Gln Gly Pro Gly Gln Gln Gly Pro Tyr Gly Pro Gly
    530                 535                 540

Ala Ser Ala Ala Ala Ala Ala Gly Gln Tyr Gly Ser Gly Pro Gly Gln
545                 550                 555                 560

Gln Gly Pro Tyr Gly Pro Gly Gln Ser Gly Ser Gly Gln Gln Gly Pro
                565                 570                 575

Gly Gln Gln Gly Pro Tyr Ala Ser Ala Ala Ala Ala Ala Gly Pro Gly
            580                 585                 590

Ser Gly Gln Gln Gly Pro Gly Ala Ser
        595                 600

<210>  2
<211>  590
<212>  PRT
<213>  Artificial Sequence

<220>
<223>  Met-PRT410

<400>  2

Met Gly Pro Gly Gln Gln Gly Pro Tyr Gly Pro Gly Ala Ser Ala Ala
1               5                   10                  15

Ala Ala Ala Gly Gln Asn Gly Pro Gly Ser Gly Gln Gln Gly Pro Gly
            20                  25                  30

Gln Ser Gly Gln Tyr Gly Pro Gly Gln Gln Gly Pro Gly Gln Gln Gly
        35                  40                  45

Pro Gly Ser Ser Ala Ala Ala Ala Ala Gly Pro Gly Gln Tyr Gly Pro
    50                  55                  60

Gly Gln Gln Gly Pro Ser Ala Ser Ala Ala Ala Ala Ala Gly Pro Gly
65                  70                  75                  80

Ser Gly Gln Gln Gly Pro Gly Ala Ser Gly Gln Tyr Gly Pro Gly Gln
                85                  90                  95

Gln Gly Pro Gly Gln Gln Gly Pro Gly Ser Ser Ala Ala Ala Ala Ala
            100                 105                 110

Gly Gln Tyr Gly Ser Gly Pro Gly Gln Gln Gly Pro Tyr Gly Ser Ala
        115                 120                 125

Ala Ala Ala Ala Gly Pro Gly Ser Gly Gln Tyr Gly Gln Gly Pro Tyr
    130                 135                 140

Gly Pro Gly Ala Ser Gly Pro Gly Gln Tyr Gly Pro Gly Gln Gln Gly
145                 150                 155                 160

Pro Ser Ala Ser Ala Ala Ala Ala Ala Gly Ser Gly Gln Gln Gly Pro
                165                 170                 175

Gly Gln Tyr Gly Pro Tyr Ala Ser Ala Ala Ala Ala Ala Gly Gln Tyr
            180                 185                 190

Gly Ser Gly Pro Gly Gln Gln Gly Pro Tyr Gly Pro Gly Gln Ser Gly
        195                 200                 205

Ser Gly Gln Gln Gly Pro Gly Gln Gln Gly Pro Tyr Ala Ser Ala Ala
    210                 215                 220

Ala Ala Ala Gly Pro Gly Gln Gln Gly Pro Tyr Gly Pro Gly Ser Ser
225                 230                 235                 240

Ala Ala Ala Ala Ala Gly Gln Tyr Gly Tyr Gly Pro Gly Gln Gln Gly
                245                 250                 255

Pro Tyr Gly Pro Gly Ala Ser Gly Gln Asn Gly Pro Gly Ser Gly Gln

            260                 265                 270

Tyr Gly Pro Gly Gln Gln Gly Pro Gly Gln Ser Ala Ala Ala Ala Ala
        275                 280                 285

Gly Pro Gly Gln Gln Gly Pro Tyr Gly Pro Gly Ala Ser Ala Ala Ala
    290                 295                 300

Ala Ala Gly Gln Tyr Gly Pro Gly Gln Gln Gly Pro Gly Gln Tyr Gly
305                 310                 315                 320

Pro Gly Ser Ser Gly Pro Gly Gln Gln Gly Pro Tyr Gly Pro Gly Ser
                325                 330                 335

Ser Ala Ala Ala Ala Ala Gly Gln Tyr Gly Pro Gly Gln Gln Gly Pro
            340                 345                 350

Tyr Gly Pro Gly Gln Ser Ala Ala Ala Ala Ala Gly Gln Tyr Gln Gln
        355                 360                 365

Gly Pro Gly Gln Gln Gly Pro Tyr Gly Pro Gly Ala Ser Gly Pro Gly
    370                 375                 380

Gln Gln Gly Pro Tyr Gly Pro Gly Ala Ser Ala Ala Ala Ala Ala Gly
385                 390                 395                 400

Pro Gly Gln Tyr Gly Pro Gly Gln Gln Gly Pro Ser Ala Ser Ala Ala
                405                 410                 415

Ala Ala Ala Gly Gln Tyr Gly Ser Gly Pro Gly Gln Tyr Gly Pro Tyr
            420                 425                 430

Gly Pro Gly Gln Ser Gly Pro Gly Ser Gly Gln Gln Gly Gln Gly Pro
        435                 440                 445

Tyr Gly Pro Gly Ala Ser Ala Ala Ala Ala Ala Gly Gln Tyr Gly Pro
    450                 455                 460

Gly Gln Gln Gly Pro Tyr Gly Pro Gly Gln Ser Ala Ala Ala Ala Ala
465                 470                 475                 480

Gly Pro Gly Ser Gly Gln Tyr Gly Pro Gly Ala Ser Gly Gln Asn Gly
                485                 490                 495

Pro Gly Ser Gly Gln Tyr Gly Pro Gly Gln Gln Gly Pro Gly Gln Ser
            500                 505                 510

Ala Ala Ala Ala Ala Gly Gln Tyr Gln Gln Gly Pro Gly Gln Gln Gly
        515                 520                 525

Pro Tyr Gly Pro Gly Ala Ser Ala Ala Ala Ala Ala Gly Gln Tyr Gly
    530                 535                 540

Ser Gly Pro Gly Gln Gln Gly Pro Tyr Gly Pro Gly Gln Ser Gly Ser
545                 550                 555                 560

Gly Gln Gln Gly Pro Gly Gln Gln Gly Pro Tyr Ala Ser Ala Ala Ala
                565                 570                 575

Ala Ala Gly Pro Gly Ser Gly Gln Gln Gly Pro Gly Ala Ser
            580                 585                 590

<210>  3
<211>  12
<212>  PRT
<213>  Artificial Sequence

<220>
<223>  HisTag

<400>  3

Met His His His His His His Ser Ser Gly Ser Ser
1               5                   10

<210>  4
<211>  601
<212>  PRT
<213>  Artificial Sequence

<220>
<223>  PRT918

<400>  4
   Met His His His His His His Ser Ser Gly Ser Ser Gly Pro Gly Val
   1               5                   10                  15
   Phe Gly Pro Tyr Gly Pro Gly Ala Ser Ala Ala Ala Ala Ala Gly Ile
               20                  25                  30
   Asn Gly Pro Gly Ser Gly Val Phe Gly Pro Gly Ile Ser Gly Ile Tyr
           35                  40                  45
   Gly Pro Gly Val Phe Gly Pro Gly Val Phe Gly Pro Gly Ser Ser Ala
       50                  55                  60
   Ala Ala Ala Ala Gly Pro Gly Ile Tyr Gly Pro Gly Val Phe Gly Pro
   65                  70                  75                  80
   Ser Ala Ser Ala Ala Ala Ala Ala Gly Pro Gly Ser Gly Val Phe Gly
   85 90 95
   Pro Gly Ala Ser Gly Ile Tyr Gly Pro Gly Val Phe Gly Pro Gly Val
   100 105 110
   Phe Gly Pro Gly Ser Ser Ala Ala Ala Ala Ala Gly Ile Tyr Gly Ser
   115 120 125
   Gly Pro Gly Val Phe Gly Pro Tyr Gly Ser Ala Ala Ala Ala Ala Gly
   130 135 140
   Pro Gly Ser Gly Ile Tyr Gly Ile Gly Pro Tyr Gly Pro Gly Ala Ser
   145 150 155 160
   Gly Pro Gly Ile Tyr Gly Pro Gly Val Phe Gly Pro Ser Ala Ser Ala
   165 170 175
   Ala Ala Ala Ala Gly Ser Gly Val Phe Gly Pro Gly Ile Tyr Gly Pro
   180 185 190
   Tyr Ala Ser Ala Ala Ala Ala Ala Gly Ile Tyr Gly Ser Gly Pro Gly
   195 200 205
   Val Phe Gly Pro Tyr Gly Pro Gly Ile Ser Gly Ser Gly Val Phe Gly
   210 215 220
   Pro Gly Val Phe Gly Pro Tyr Ala Ser Ala Ala Ala Ala Ala Gly Pro
   225 230 235 240
   Gly Val Phe Gly Pro Tyr Gly Pro Gly Ser Ser Ala Ala Ala Ala Ala
   245 250 255
   Gly Ile Tyr Gly Tyr Gly Pro Gly Val Phe Gly Pro Tyr Gly Pro Gly
   260 265 270
   Ala Ser Gly Ile Asn Gly Pro Gly Ser Gly Ile Tyr Gly Pro Gly Val
   275 280 285
   Phe Gly Pro Gly Ile Ser Ala Ala Ala Ala Ala Gly Pro Gly Val Phe
   290 295 300
   Gly Pro Tyr Gly Pro Gly Ala Ser Ala Ala Ala Ala Ala Gly Ile Tyr
   305 310 315 320
   Gly Pro Gly Val Phe Gly Pro Gly Ile Tyr Gly Pro Gly Ser Ser Gly
   325 330 335
   Pro Gly Val Phe Gly Pro Tyr Gly Pro Gly Ser Ser Ala Ala Ala Ala
               340                 345                 350
   Ala Gly Ile Tyr Gly Pro Gly Val Phe Gly Pro Tyr Gly Pro Gly Ile
           355                 360                 365
   Ser Ala Ala Ala Ala Ala Gly Ile Tyr Val Phe Gly Pro Gly Val Phe
       370                 375                 380
   Gly Pro Tyr Gly Pro Gly Ala Ser Gly Pro Gly Val Phe Gly Pro Tyr
   385                 390                 395                 400
   Gly Pro Gly Ala Ser Ala Ala Ala Ala Ala Gly Pro Gly Ile Tyr Gly
                   405                 410                 415
   Pro Gly Val Phe Gly Pro Ser Ala Ser Ala Ala Ala Ala Ala Gly Ile
               420                 425                 430
   Tyr Gly Ser Gly Pro Gly Ile Tyr Gly Pro Tyr Gly Pro Gly Ile Ser
           435                 440                 445
   Gly Pro Gly Ser Gly Val Phe Gly Ile Gly Pro Tyr Gly Pro Gly Ala
       450                 455                 460
   Ser Ala Ala Ala Ala Ala Gly Ile Tyr Gly Pro Gly Val Phe Gly Pro
   465                 470                 475                 480
   Tyr Gly Pro Gly Ile Ser Ala Ala Ala Ala Ala Gly Pro Gly Ser Gly
                   485                 490                 495
   Ile Tyr Gly Pro Gly Ala Ser Gly Ile Asn Gly Pro Gly Ser Gly Ile
               500                 505                 510
   Tyr Gly Pro Gly Val Phe Gly Pro Gly Ile Ser Ala Ala Ala Ala Ala
           515                 520                 525
   Gly Ile Tyr Val Phe Gly Pro Gly Val Phe Gly Pro Tyr Gly Pro Gly
       530                 535                 540
   Ala Ser Ala Ala Ala Ala Ala Gly Ile Tyr Gly Ser Gly Pro Gly Val
   545                 550                 555                 560
   Phe Gly Pro Tyr Gly Pro Gly Ile Ser Gly Ser Gly Val Phe Gly Pro
                   565                 570                 575
   Gly Val Phe Gly Pro Tyr Ala Ser Ala Ala Ala Ala Ala Gly Pro Gly
   580                 585                 590
   Ser Gly Val Phe Gly Pro Gly Ala Ser
           595                 600

<210>  5
<211>  590
<212>  PRT
<213>  Artificial Sequence

<220>
<223>  Met-PRT918

<400>  5

Met Gly Pro Gly Val Phe Gly Pro Tyr Gly Pro Gly Ala Ser Ala Ala
1               5                   10                  15

Ala Ala Ala Gly Ile Asn Gly Pro Gly Ser Gly Val Phe Gly Pro Gly
            20                  25                  30

Ile Ser Gly Ile Tyr Gly Pro Gly Val Phe Gly Pro Gly Val Phe Gly
        35                  40                  45

Pro Gly Ser Ser Ala Ala Ala Ala Ala Gly Pro Gly Ile Tyr Gly Pro
    50                  55                  60

Gly Val Phe Gly Pro Ser Ala Ser Ala Ala Ala Ala Ala Gly Pro Gly
65                  70                  75                  80

Ser Gly Val Phe Gly Pro Gly Ala Ser Gly Ile Tyr Gly Pro Gly Val
                85                  90                  95

Phe Gly Pro Gly Val Phe Gly Pro Gly Ser Ser Ala Ala Ala Ala Ala
            100                 105                 110

Gly Ile Tyr Gly Ser Gly Pro Gly Val Phe Gly Pro Tyr Gly Ser Ala
        115                 120                 125

Ala Ala Ala Ala Gly Pro Gly Ser Gly Ile Tyr Gly Ile Gly Pro Tyr
    130                 135                 140

Gly Pro Gly Ala Ser Gly Pro Gly Ile Tyr Gly Pro Gly Val Phe Gly
145                 150                 155                 160

Pro Ser Ala Ser Ala Ala Ala Ala Ala Gly Ser Gly Val Phe Gly Pro
                165                 170                 175

Gly Ile Tyr Gly Pro Tyr Ala Ser Ala Ala Ala Ala Ala Gly Ile Tyr
            180                 185                 190

Gly Ser Gly Pro Gly Val Phe Gly Pro Tyr Gly Pro Gly Ile Ser Gly
        195                 200                 205

Ser Gly Val Phe Gly Pro Gly Val Phe Gly Pro Tyr Ala Ser Ala Ala
    210                 215                 220

Ala Ala Ala Gly Pro Gly Val Phe Gly Pro Tyr Gly Pro Gly Ser Ser
225                 230                 235                 240

Ala Ala Ala Ala Ala Gly Ile Tyr Gly Tyr Gly Pro Gly Val Phe Gly
                245                 250                 255

Pro Tyr Gly Pro Gly Ala Ser Gly Ile Asn Gly Pro Gly Ser Gly Ile
            260                 265                 270

Tyr Gly Pro Gly Val Phe Gly Pro Gly Ile Ser Ala Ala Ala Ala Ala
        275                 280                 285

Gly Pro Gly Val Phe Gly Pro Tyr Gly Pro Gly Ala Ser Ala Ala Ala
    290                 295                 300

Ala Ala Gly Ile Tyr Gly Pro Gly Val Phe Gly Pro Gly Ile Tyr Gly
305                 310                 315                 320

Pro Gly Ser Ser Gly Pro Gly Val Phe Gly Pro Tyr Gly Pro Gly Ser
                325                 330                 335

Ser Ala Ala Ala Ala Ala Gly Ile Tyr Gly Pro Gly Val Phe Gly Pro
            340                 345                 350

Tyr Gly Pro Gly Ile Ser Ala Ala Ala Ala Ala Gly Ile Tyr Val Phe
        355                 360                 365

Gly Pro Gly Val Phe Gly Pro Tyr Gly Pro Gly Ala Ser Gly Pro Gly
    370                 375                 380

Val Phe Gly Pro Tyr Gly Pro Gly Ala Ser Ala Ala Ala Ala Ala Gly
385                 390                 395                 400

Pro Gly Ile Tyr Gly Pro Gly Val Phe Gly Pro Ser Ala Ser Ala Ala
                405                 410                 415

Ala Ala Ala Gly Ile Tyr Gly Ser Gly Pro Gly Ile Tyr Gly Pro Tyr
            420                 425                 430

Gly Pro Gly Ile Ser Gly Pro Gly Ser Gly Val Phe Gly Ile Gly Pro

435                 440                 445

Tyr Gly Pro Gly Ala Ser Ala Ala Ala Ala Ala Gly Ile Tyr Gly Pro
    450                 455                 460

Gly Val Phe Gly Pro Tyr Gly Pro Gly Ile Ser Ala Ala Ala Ala Ala
465                 470                 475                 480

Gly Pro Gly Ser Gly Ile Tyr Gly Pro Gly Ala Ser Gly Ile Asn Gly
                485                 490                 495

Pro Gly Ser Gly Ile Tyr Gly Pro Gly Val Phe Gly Pro Gly Ile Ser
            500                 505                 510

Ala Ala Ala Ala Ala Gly Ile Tyr Val Phe Gly Pro Gly Val Phe Gly
        515                 520                 525

Pro Tyr Gly Pro Gly Ala Ser Ala Ala Ala Ala Ala Gly Ile Tyr Gly
    530                 535                 540

Ser Gly Pro Gly Val Phe Gly Pro Tyr Gly Pro Gly Ile Ser Gly Ser
545                 550                 555                 560

Gly Val Phe Gly Pro Gly Val Phe Gly Pro Tyr Ala Ser Ala Ala Ala
                565                 570                 575

Ala Ala Gly Pro Gly Ser Gly Val Phe Gly Pro Gly Ala Ser
            580                 585                 590